# EUROPEAN PATENT APPLICATION

(11) **EP 2 982 764 A1**
(43) Date of publication of application: **10.02.2016**
(21) Application number: 14179794.4
(22) Date of filing: 05.08.2014
(51) Int. Cl.: C12Q 1/68

(54) **Identification of vaginal bacteria**

(71) Applicant: ALFA WASSERMANN S.p.A., 65020 Alanno (PE) (IT)
(72) Inventor: Viscomi, Giuseppe Claudio, 40133 Bologna (IT); Calanni, Fiorella, 40133 Bologna (BO) (IT); Brigidi, Patrizia, 40126 Bologna (BO) (IT); Vitali, Beatrice, 40127 Bologna (BO) (IT); Biagi, Elena, 40126 Bologna (BO) (IT); Cruciani, Federica, 40127 Bologna (BO) (IT); Severgnini, Marco, 20090 Segrate (MI) (IT); Consolandi, Clarissa, 20090 Segrate (MI) (IT)

(57) **Abstract**

The present invention relates to a method for diagnosing vaginal infections and for evaluating the efficacy of treatment of vaginal infection detecting the presence or absence of one or more vaginal bacteria in a vaginal fluid sample. The method is also useful for evaluating the relapse after the end of antibiotic treatment.

## Description

### Abstract

The present invention relates to a method for diagnosing vaginal infections and for evaluating the efficacy of treatment of vaginal infection detecting the presence or absence of one or more vaginal bacteria in a vaginal fluid sample. The method is also useful for evaluating the relapse after the end of antibiotic treatment.

### Background

Traditional methods for the identification of microorganisms are based on body fluid culture methods, requiring the microbial cultivation with subsequent morphological and physiological characterization. Traditional identification methods for microorganisms in everyday clinical life are usually based on time-consuming procedures with morphological and physiological characterizations. Body fluid culture methods are the gold-standard in the diagnosing of microbial infections and early identification of infection-causing microbes is the crucial requisite for a fast and optimally targeted infection treatment.

Unfortunately, the conventional diagnostic methods need at least 24 hours for obtaining results due to their requirement for microbial enrichment and are unable to detect the so-called "viable not-culturable" organisms.

Many diagnostic methods are based on bacterial quantification in blood stream based on spreading of whole blood and subsequent evaluation by counting the colony forming units (CFU). In general the detection and their identification is a lengthy process usually ranging from 2 to 5 days for most organisms or even longer for fastidious organisms as described by Marlowe et al in J Clin Microbial, 2003, 41, 5127.

Methods based on PCR amplification and subsequent hybridization of fluorescent probes seem to be the most promising approaches for diagnosing disease by body fluid as described by Peters et al in Lancet Infect Dis. 2004,4(12),751.

Fluorescent in situ hybridization, PCR, Real time PCR, fluorescence-based PCR-single strand conformation polymorphism (SSCP) and oligonucleotide microarrays have been employed for the identification of microorganisms from patients including a cultivation-based bacterial enrichment step.

Microarray technologies have been described as a powerful tool for various clinical applications such a pathogen identification of urinary tract infections, acute upper respiratory tract infections, periodontal pathogens and intestinal bacteria. Microarrays are further applied for the analysis of microbial gene expression and diversity. Bacterial vaginosis is an extremely frequent pathology, representing 40-50% of all vaginal infections. When it is symptomatic and without complications, bacterial vaginosis is characterized by malodorous vaginal discharges not associated to an inflammatory clinical picture (vaginosis), and is attributed to an alteration of the vaginal ecosystem.

The normal vaginal flora of a healthy woman for the presence of *Lactobacillus,* in particular *Lactobacillus crispatus* and *Lactobacillus gasseri,* produces hydrogen peroxide and maintains an acid vaginal pH, thus inhibiting the growth of most pathogenic microorganisms.

In bacterial vaginosis, lactobacilli are replaced by an excessive growth, even a thousand times higher than normal values, of facultative anaerobic and aerobic bacteria, mainly represented by *Gardnerella vaginalis,* which is present in nearly all women affected by bacterial vaginosis, by *Mycoplasma hominis,* by Gram-negative anaerobic bacteria such as *Bacteroides* and *Prevotella,* by anaerobes such as *Peptostreptococcus,* by Gram-positive anaerobes such as *Mobiluncus,* which is present in 50% of the cases, and by Gram-positive bacilli such as *Atopobium vaginale,* which is present in 95% of cases of bacterial vaginosis.

Since bacterial vaginosis etiology is not completely understood, the clinical treatments have the aim of inducing both a clinical and a microbiological recovery and, when possible, avoiding the relapse infections. An ideal therapy should be effective at reducing pathogenic species and, at the same time, it should also encourage the restoration and proliferation of *Lactobacillus* protective species with the aim of preventing possible disease relapses.

Treatment of vaginal infection usually foresees the administration of antibiotic and/or antibiotic composition by oral or vaginal route.

WO 2013/017928 describes compositions comprising rifaximin in form of vaginal tablets and their use in treatment of vaginal infections. WO 2013/017928 also describes efficacious dosage treatment and treatment time to obtain remission of the disease without relapse.

Rifaximin (INN, see The Merck Index, XIII ed., 8304, CAS No.80621-81-4), IUPAC nomenclature 2S, 16Z, 18E, 20S, 21S, 22R, 23R, 24R, 25S, 26S, 27S, 28E)-5,6,21,23,25 pentahydroxy-27-methoxy-2,4,11,16,20, 22,24,26-octamethyl-2,7-(epoxypentadeca (1,11,13) trienimine) benzofuro (4,5-e) pyrido(1,2,-a benzimidazole-1,15(2H)dione, 25-acetate) is a semysinthetic antibiotic drug belonging to the rifampicin group, more precisely a pyrido-imidazo-rifamycin described in IT 1154655, whereas EP 0 161 534 describes a production process starting from Rifamycin O (The Merck Index XIII ed., 8301).Rifaximin can be in polymorphous, amorphous, hydrate or solvate forms e.g. US 7,045,620, EP 1557421 B1, EP 1676847B1, EP 1676848B1, WO2005/044823, WO2006/094662 describe crystalline forms α, β, γ, δ and ε of rifaximin. WO2008/155728, US 2009/312357 and US2008/008253 describe processes for obtaining amorphous forms.

The diagnosis of bacterial vaginosis and the evaluation of treatment efficacy is usually based upon clinical and/or microbiological criteria.

Clinical diagnosis is carried out according to Amsel clinical criteria, as described by Amsel R. et al. in Am. J. Med. 1983; 74(1): 14-22. The diagnosis is positive when at least three out of the four following symptoms are reported: 1) vaginal discharges which are homogeneous and adhering to the vaginal walls; 2) whiff test positivity (development of "fishy odor" after the addition of 10% potassium hydroxide to vaginal discharge); 3) vaginal pH higher than 4.5, and 4) an amount greater than 20% of clue cells (squamous epithelium vaginal cells coated with bacteria, identified by fresh microscopic examination).

The microbiological diagnosis is based on the calculation of the Nugent score, which includes microscopic examination of vaginal discharges by means of Gram staining. The presence and the quantity of three different vaginal bacterial species is determined. In particular, a low score is obtained if the Lactobacilli concentration is high, the score increases if the presence of *Gardnerella* and *Bacteroides* is ascertained, and the score is even higher if also the presence of *Mobiluncus* is ascertained. A resulting score between 0 and 3 is representative of vaginal flora of a healthy woman, a score between 4 and 6 indicates that vaginal flora is starting to be altered, and a score between 7 and 10 indicates a certain diagnosis of bacterial vaginosis, as described by Nugent RP et al. in J. Clin. Microbiol. 1991, 29(2), 297-301.

In recent years further diagnostic molecular techniques have been developed, such as PCR-DGGE and real-time PCR, based upon the sequence analysis of rRNA gene and allowing the identification of a microbial composition of the vaginal ecosystem, as described by Zhou X et al. in Microbiology 2004, 150 (Pt8), 2565-2573 and in Appl. Environ. Microbiol. 2004, 70(6), 3575-3581.

These techniques can be directly used for determining the presence of pathogenic agents causing the disease as diagnostic method. The main disadvantage of the PCR technique is that only one target for each sample can be analyzed. US2004/00223209 describes a primer extension reaction to visualize sequences of microorganisms for their identifications and 16S and 18S rRNA sequences can be used as probes. It discloses a method for identifying microbial pathogens in body fluids, in particular based on the reaction on the microbial DNA encoding 16S or 18S rRNA. In particular it describes a method for identifying pathogens of vaginosis in vaginal fluid samples which comprises the step of lysing the microbial pathogens and performing a nucleic acid amplification reaction on the microbial DNA encoding 16S or 18S rRNA; contacting the labeled amplified nucleic acid with a microarray comprising immobilized probes for microbial DNA encoding 16S or 18S rRNA. US2009/0291854 describes a method for the identification of microbial pathogens in a body fluid sample using the microarray wherein the DNA chip comprises immobilized probes for microbial DNA encoding 16S or 18S rRNA from the relevant pathogens of human body fluids. The methods comprises a step of performing acid nucleic amplification reaction on the microbial DNA encoding 16S and 18S rRNA wherein the amplified nucleic acids are labelled and another step wherein the labelled amplified nucleic acids are in contact with the microarray. At the end the method foresees detecting the binding of one or more species of labelled amplified nucleic acids to a probe.

These technique have not been employed to evaluate the efficacy of treatment of vaginal infection.

In the microarray format, the most used procedure is the differential hybridization of a fluorescently labelled target, often a PCR product, with microarray immobilized oligonucleotide probes. However this method requires very careful probe design and a non-trivial optimization of the experimental set up. Since the detection principle of the assay is based on oligonucleotide hybridization, the melting temperature at which the reaction occurs is fundamental. When the targets have very similar sequences, this task becomes particularly difficult and can incur in aspecific matches between the probes and the intended targets.

An early identification of infection is the crucial requisite for a fast and optimally targeted infection treatment. Moreover the availability of a tool which can allow to select the most efficacious therapy can result very helpful for a fast healing process. Therefore there was a need to develop a method reliable, fast and low-cost for firstly identification of infection caused by microorganism and secondly useful for evaluating the efficacy of treatment of said infection.

The object of the present invention is a method to identify bacterial species connected in vaginal fluid, especially pathogens being related or connected or causing vaginal infection and bacteria belonging to the healthy vaginal eco-system analyzing many sample simultaneously and multiple targets in the same sample. The present invention relates to a method able to identify the presence of vaginal infections and evaluate the efficacy of treatment of vaginal infection through the quantitative and/or qualitative determination of a 16S rRNA gene or fragment thereof derived from a vaginal bacterium and their alterations which depend on the presence of infection and/or the recovery from the disease.

The present invention describes the use of probes applied on a DNA-microarray implemented with ligase detection reaction useful for the diagnosis of vaginal infections and for the evaluation of the efficacy of the treatment of vaginal infections with antibiotics. The method is selective and sensitive determining the variations of the most representative bacterial groups that compose the vaginal microbiota, in particular of the pathogens bacteria causing the vaginal infections and also bacteria belonging to the healthy vaginal flora as *Lactobacillus.*

The method of the present invention has been employed for the evaluation of recovery in patients affected by vaginal infection and treated with rifaximin as described in WO 2013/017928.

### Summary of the invention

The present invention relates to a method for identifying the presence of vaginal infections and for evaluating the efficacy of treatment of vaginal infection.

The present invention relates to a method involving probes for targeting specified microbial species applied in a DNA-microarray implemented with ligase detection reaction (LDR) useful for the diagnosis of vaginal infection and for evaluating the efficacy of treatment of vaginal infection.

The method is able to detect the presence or absence of one or more vaginal bacteria in a vaginal fluid sample and evaluating the efficacy of treatment of vaginal infection with antibiotic.

The present invention relates to the use of probes in a DNA-microarray implemented with LDR.

The present invention relates to a kit for detecting the presence or absence of one or more vaginal bacteria in a vaginal fluid sample.

The present invention relates to the use of a kit for diagnosing a vaginal infection and for evaluating the efficacy of treatment during the treatment and the relapse after the end of the treatment of a vaginal infection.

The present invention relates to the use of a kit for determining an efficacious antibiotic dosage in the treatment of a vaginal infection.

The present invention relates to a set of oligonucleotides specific for targeting a nucleic acid encoding 16S rRNA of a vaginal bacterium.

According to the present invention, there is provided a method of detecting the presence or absence of one or more vaginal bacteria in a vaginal fluid sample comprising the steps of
a) providing a vaginal fluid sample,
b) extracting the bacterial DNA from the bacteria contained in the sample,
c) performing a nucleic acid amplification reaction on the microbial DNA encoding 16S rRNA,
d) contacting the amplified nucleic acids from step c) with a pair of two adjacent probes specific for each bacterial species, a discriminating probe comprising a 5'-fluorophore-modified oligonucleotide, and a common probe starting one base 3'-downstream of the discriminating probe and comprising a 5'-phosphate group and a unique sequence named cZipcode at its 3'-end, to obtain a paired mix of the two probes,
e) subjecting the paired mix of step d) to a thermostable DNA ligase reaction in a thermal cyclic reaction with PCR amplicons as templates,
f) contacting the ligated fragments of step e) with a universal DNA array on which the unique Zipcode sequences have been spotted,
g) detecting the binding of one or more ligated fragments by laser scanning of the array,
h) identifying the presence or absence of one or more vaginal bacteria by correlating the detected binding of the ligated fragments with the defined area of the unique cZipcode sequence immobilized on the array by laser scanning,
wherein a set of probes are used comprising
- probe pairs designed for targeting a nucleic acid encoding 16S rRNA of a Lactobacillus species selected in the group comprising *L. crispatus, L. iners, L. vaginalis, L. acidophilus, L. gasseri* et rel. *(L. gasseri* and *L. johnsonii)* and *L. jensenii* et rel. *(L. jensenii* and *L. fornicalis*); and
- probe pairs designed for targeting a nucleic acid encoding 16S rRNA of a pathogen bacteria selected from the group comprising *Atopobium vaginae* and *Mycoplasma hominis,* the *Sneathia* group *(S. sanguinegens* and S. *amnii,* formerly *Leptotrichia amnionii),* the genera *Streptococcus, Staphylococcus, Veillonella, Megasphaera, Mobiluncus, Leptotrichia and Eggerthella, Bacteroides Prevotella.*

According to one aspect the probes are used comprising
- a first probe pairs designed for targeting a nucleic acid encoding 16S rRNA of a *Lactobacillus* species selected in the group comprising *L. crispatus, L. iners, L. vaginalis, L. acidophilus, L. gasseri* et rel. *(L. gasseri* and *L. johnsonii)* and *L. jensenii* et rel. *(L. jensenii* and *L. fornicalis*); and
- a second probe pairs designed for targeting a nucleic acid encoding 16S rRNA of a pathogen bacteria selected from the group comprising *Atopobium vaginae* and *Mycoplasma hominis,* the *Sneathia* group *(S. sanguinegens* and S. *amnii,* formerly *Leptotrichia amnionii),* the genera *Streptococcus, Staphylococcus, Veillonella, Megasphaera, Mobiluncus, Leptotrichia and Eggerthella, Bacteroides Prevotella.* The invention further provides a kit for detecting the presence or absence of one or more vaginal bacteria in a vaginal fluid sample, comprising
   i) a microarray on which a plurality of specific recognition elements containing one or more unique Zipcode sequences have been immobilized in discrete measurement areas of known location,
   ii) a sample holding means for a vaginal sample,
   iii) a discriminating probe comprising a 5'-fluorophore-modified oligonucleotide specific for a vaginal bacterium and being designed for targeting 16S rRNA of *Lactobacillus* species selected in the group comprising *L. crispatus, L. iners, L. vaginalis, L. acidophilus, L. gasseri* et rel. *(L. gasseri* and *L. johnsonii)* and *L. jensenii* et rel. *(L. jensenii* and *L. fornicalis*) or of pathogen bacteria selected from the group comprising *Atopobium vaginae* and *Mycoplasma hominis,* the *Sneathia* group (S. *sanguinegens* and S. *amnii,* formerly *Leptotrichia amnionii*), the genera *Streptococcus, Staphylococcus, Veillonella, Megasphaera, Mobiluncus, Leptotrichia and Eggerthella, Bacteroides Prevotella,*
   iv) a common probe specific for a vaginal bacterium, starting one base 3'-downstream of the discriminating probe and comprising a 5'-phosphate group and a unique sequence named cZipcode at its 3'-end and being designed for targeting 16S rRNA of Lactobacillus species selected in the group comprising *L. crispatus, L. iners, L. vaginalis, L. acidophilus, L. gasseri* et rel. *(L. gasseri* and *L. johnsonii*) and *L*. *jensenii* et rel. *(L. jensenii* and *L. fornicalis*) or of pathogen bacteria selected from the group comprising *Atopobium vaginae* and *Mycoplasma hominis,* the *Sneathia* group *(S. sanguinegens* and S. *amnii,* formerly *Leptotrichia amnionii),* the genera *Streptococcus, Staphylococcus, Veillonella, Megasphaera, Mobiluncus, Leptotrichia* and *Eggerthella,* Bacteroides Prevotella, and
   v) a ligation mix.

The invention further provides a set of oligonucleotides, characterized in that the set comprises a first probe selected from SEQ ID NOs: 1 to 17 and a second probe selected from SEQ ID NOs: 18 to 34, whereby the first and the second probe are specific for the same organism.

The invention further provides a set of oligonucleotides specific for targeting a nucleic acid encoding 16S rRNA of a vaginal bacterium, characterized in that the set comprises oligonucleotides specific for targeting a nucleic acid encoding 16S rRNA of *Lactobacillus* species selected in the group comprising *L. crispatus, L. iners, L. vaginalis, L. acidophilus, L. gasseri* et rel. *(L. gasseri* and *L. johnsonii)* and *L. jensenii* et rel. *(L. jensenii* and *L. fornicalis*); and
oligonucleotides specific for targeting a nucleic acid encoding 16S rRNA of pathogen bacteria selected in the group comprising *Atopobium vaginae* and *Mycoplasma hominis,* the *Sneathia* group (*S. sanguinegens* and *S*. *amnii,* formerly *Leptotrichia amnionii),* the genera *Streptococcus, Staphylococcus, Veillonella, Megasphaera, Mobiluncus, Leptotrichia* and *Eggerthella, Bacteroides Prevotella.*

### Detailed description of the invention

The present invention describes a fast, reliable and low-cost method of analysis which is able to screen many samples in a short time, useful for the identification of pathogens in women affected by vaginal infections and for the evaluation of efficacy of treatment of vaginal infections.

The method of diagnosis object of the present invention is useful for determining an efficacious antibiotic dosage for treating vaginal infection and for evaluating the relapse after the end of the treatment.

The present invention relates to a method involving probes for targeting specified microbial species applied in a DNA-microarray useful for the diagnosis of vaginal infection, and for evaluating the efficacy of antibiotic in the treatment of vaginal infection. Preferably the vaginal infection is bacterial vaginosis and the antibiotic is rifaximin.

The present invention relates to a method involving probes for targeting *Lactobacillus* species applied in a DNA-microarray for determining the amount of *Lactobacillus* in vaginal fluid which are indicative of healthy vaginal eco-system.

The method of the present invention can be used also in the prevention screening of vaginal infection evaluating if the patients can relapse after treatment.

According to one aspect, the present invention relates to a method of diagnosing of vaginal infection and for evaluating the efficacy of treatment involving a universal DNA-microarray, a phylogenetic DNA-microarray designed for the taxonomic fingerprint of the human vaginal microbiota, implemented with ligase detection reaction (LDR) which comprises two probes specific for each target sequence, one oligo carries a fluorescent label named discriminating probe (DP) and the other a unique sequence (complementary ZipCode) named common probe (CP). The ligated fragments are obtained in presence of template 16S rRNA of selected bacteria groups or species of particular relevance for the vaginal microbial ecosystem.

The method of the present invention involves probe pairs for targeting specified microbial species applied in a DNA-microarray wherein the probes pairs were designed using 16S rRNA sequences available in the Ribosomial Database Project (RDP) of the selected bacterial groups or species present in vaginal fluid.

The vaginal bacterial groups that has been found to be most representative of the vaginal microbiota of healthy woman and affected by bacterial vaginosis have been selected. For each selected target a positive and negative set of sequences have been created.

The best probe pairs have been selected for each target on the basis of specificity profiles obtained by the comparison with the sequences available in the database. The selection of discriminating position which differs a target respect to the others non target species is not obvious since many targets have a high similarity among them, wherein said similarity can reach (for the 16S rRNA region) a value greater of 87% and up to 98%, as occurred for Lactobacillaceae families. The discriminating position and also the entire probes sequences, has not to match with no one of the non targeting sequences. For the above reason a complete negative set is necessary for the specific contest.

The identification of the discriminating position which would be present in the consensus sequence of all or most of selected strains of the same species constituting the positive set, represents a crucial aspect of the method.

The design has been developed on the basis of thermodynamic parameters as melting temperature of about 68°C, in order to minimize the possible interaction between probes and avoiding an aspecific annealing of the probes on a target template which can reduce the specificity of the array.

According to one aspect of the invention, the probe pairs, DP and CP, were designed for targeting the species associated to the altered ecosystem typical of bacterial vaginosis such as *Atopobium vaginae* and *Mycoplasma hominis,* the Sneathia group (*S. sanguinegens and S. amnii, formerly Leptotrichia amnionii*), the genera *Streptococcus, Staphylococcus, Veillonella, Megasphaera, Mobiluncus, Leptotrichia* and *Eggerthella, Bacteroides Prevotella.*

In one aspect the probe pairs, DP and CP, were designed for targeting Lactobacillaceae families such as *L. crispatus, L. iners, L. vaginalis, L. acidophilus, L. gasseri* et rel. (*L. gasseri and L. johnsonii*) and *L. jensenii* et rel. *(L. jensenii* and *L*. *fornicalis*).

According to another aspect of the invention the probe pairs, DP and CP, have a length between 25 and 52 nucleotides and melting temperature comprised between 65 and 75°C.

According to another aspect of the invention the probes were designed for targeting the bacterial species reported in Table 1.

**Table 1: Probe set of microarray.**

| **Probe** | **Taxonomic level** | **Phylum** | **Specificity** |
|---|---|---|---|
| *Lactobacillus vaginalis* | Species | *Firmicutes* | *L. vaginalis* |
| *Lactobacillus iners* | Species | *Firmicutes* | *L. iners* |
| *Lactobacillus crispatus* | Species | *Firmicutes* | *L. crispatus* |
| *Lactobacillus acidophilus* | Species | *Firmicutes* | *L. acidophilus* |
| *Lactobacillus jensenii et rel.* | Closely related species | *Firmicutes* | *L. jensenii, L. fornicalis* |
| *Lactobacillus gasseri et rel.* | Closely related species | *Firmicutes* | *L. gasseri, L. johnsonii* |
| *Streptococcus* | Genus | *Firmicutes* | *Streptococcus* sp. |
| *Staphylococcus* | Genus | *Firmicutes* | *Staphylococcus* sp. |
| *Veillonella* | Genus | *Firmicutes* | *Veillonella* sp. |
| *Megasphaera* | Genus | *Firmicutes* | *Megasphaera* sp. |
| *Bacteroides*/*Prevotella* | Cluster | *Bacteroidetes* | *Prevotella* sp. |
| *Mobiluncus* | Genus | *Actinobacteria* | *Mobiluncus* sp. |
| *Atopobium vaginae* | Species | *Actinobacteria* | *A. vaginae* |
| *Eggerthella* | Genus | *Actinobacteria* | *Eggerthella* sp. |
| *Sneathia* | Genus | *Fusobacteria* | *S. sanguinegens, S. amnii* (formerly *Leptotrichia amnionii*) |
| *Leptotrichia* | Genus | *Fusobacteria* | *Leptotrichia* sp. |
| *Mycoplasma hominis* | Species | *Tenericutes* | *M. hominis* |

According to another aspect of the invention the set of oligonucleotides specific for targeting a nucleic acid encoding 16S rRNA of a vaginal bacterium, is selected from the sequences of Table 2A and 2B, wherein the table 2A reports the sequences of discriminating probes and the table 2B reports the sequences of common probes.

**Table 2A**

| **Probe Name** | **Zip code** | **Discriminating Probe (DP)** | **DS bp** | **Tm DS** | **Deg DS** |
|---|---|---|---|---|---|
| *L. vaginalis* | 1 | GCCCAAAGTCGGTGGCCTAACC ATTTGGAGGGAA (SEQ ID NO: 1) | 34 | 68.0 | 0 |
| *L. iners* | 5 | CCTGGAAACAGATGCTAATACC GGATAACAACAGATGATGCCTA (SEQ ID NO: 2) | 44 | 67.3 | 0 |
| *L. crispatus* | 9 | GAACCTTACCAGGTCTTGACAT CTAGTGCCATTTGTAGAGATAC A (SEQ ID NO: 3) | 45 | 67.3 | 0 |
| *L. jensenii* | 15 | GCAACCCTTGTTAATAGTTGCCA GCATTAAGTTGGGCACTCTAT (SEQ ID NO: 4) | 44 | 67.3 | 0 |
| *L. gasseri* | 19 | GGTAGTGAAGAAAGATAGAGGT AGTAACTGGCCTTTATTTGACG GTAATTAC (SEQ ID NO: 5) | 52 | 67.7 | 0 |
| *L. acidophilus* | 23 | GGTGCTAATACCGGATAAGAAA GCAGATCGCATGATCAGCTTA (SEQ ID NO: 6) | 43 | 67.4 | 0 |
| *Streptococcus* | 11B | CGTAAAGCGAGCGCAGGCGGT TWDATAAGTCTGAAGTT (SEQ ID NO: 7) | 38 | 67.7 | 2 |
| *Staphylococcus* | 29 | CAACCCTTAAGCTTAGTTGCCAK CATTAAGTTGGGCACTCTARGTT (SEQ ID NO: 8) | 46 | 67.7 | 1 |
| *Veillonella* | 3 | CACCGCCCGTCACACCACGAAA GTCGGA (SEQ ID NO: 9) | 28 | 67.2 | 0 |
| *Megasphaera* | 34 | CTACAATGGCTCTTAATAGAGG GAAGCGAAGGAGCGATC (SEQ ID NO: 10) | 39 | 67.6 | 0 |
| *Pre votella* | 16 | CATTAAGIATICCACCTGGGGAG TACGCCGGCAAC (SEQ ID NO: 11) | 35 | 69.1 | 2 |
| *Mobiluncus* | 1B | CGTCGCGTCTGTCGTGAAAGCC AGCAGCTTAACT (SEQ ID NO: | 34 | 68.0 | 0 |
| | | 12) | | | |
| *A. vaginae* | 23B | CCGCTCCTGATACCGGCAGGCT TGAGTC (SEQ ID NO: 13) | 28 | 67.2 | 0 |
| *Eggerthella* | 5B | CGTAGGCGGCCTCTCAAGCGG GATCTCT (SEQ ID NO: 14) | 28 | 67.2 | 0 |
| *Sneathia* | 7 | GATCCAGCAATTCTGTGTGTGT GAAGAAGGTTTTAGGACTGTAA AACA (SEQ ID NO: 15) | 48 | 67.1 | 0 |
| *Leptotrichia* | 8 | GAGGTGAAATTCGTAGATATGT GCAGGAATGCCGATGATGAAGA T (SEQ ID NO: 16) | 45 | 67.3 | 0 |
| *M. hominis* | 12 | GTAAGGCGCCACTAAAAGATGA GGGTGCGGAACATTAGT (SEQ ID NO: 17) | 39 | 67.6 | 0 |

**Table 2B**

| **Probe Name** | **Zip code** | **Common probe (CP)** | **CP bp** | **Tm CP** | **Deg CP** |
|---|---|---|---|---|---|
| *L. vaginalis* | 1 | CCGCCTAAGGCAGGGCAGATG ACTGGG (SEQ ID NO: 18) | 27 | 67.3 | 0 |
| *L. iners* | 5 | TCAACTGTTTAAAAGATGGTTCT GCTATCACTCTTGGATGGACCT G (SEQ ID NO: 19) | 46 | 67.2 | 0 |
| *L. crispatus* | 9 | AAGTTCCCTTCGGGGACGCTAA GACAGGTGGTG (SEQ ID NO: 20) | 33 | 68.1 | 0 |
| *L. jensenii* | 15 | TGAGACTGCCGGTGACAAACCG GAGGAAGG (SEQ ID NO: 21) | 30 | 67.1 | 0 |
| *L. gasseri* | 19 | TTAGAAAGTCACGGCTAACTAC GTGCCAGCAGCCG (SEQ ID NO: 22) | 35 | 67.9 | 0 |
| *L. acidophilus* | 23 | TAAAAGGCGGCGTAAGCTGTCG CTATGGGATGGC (SEQ ID NO: 23) | 34 | 68.0 | 0 |
| *Streptococcus* | 11B | AAAGGCWGTGGCTYAACCATW GTACGCTTTGGAAACTGTTAAAC | 47 | 67.2 | 3 |
| | | TTG (SEQ ID NO: 24) | | | |
| *Staphylococcus* | 29 | GACTGCCGGTGACAAACCGGA GGAAGGTG (SEQ ID NO: 25) | 29 | 67.1 | 0 |
| *Veillonella* | 3 | AGTGCCCAAAGCCGGTGGGGT AACCTTCG (SEQ ID NO: 26) | 29 | 67.1 | 0 |
| *Megasphaera* | 34 | CGGAGCAAACCCCAAAAACAGA GTCCCAGTTCGG (SEQ ID NO: 27) | 34 | 68.0 | 0 |
| *Pre votella* | 16 | GGTGAAACTCAAAGGAATTGAC GGGGGCCCGC (SEQ ID NO: 28) | 32 | 68.2 | 0 |
| *Mobiluncus* | 1B | GTTGGTCTGCGGTGGGTACGG GCGG (SEQ ID NO: 29) | 25 | 67.5 | 0 |
| *A. vaginae* | 23B | TGGTAGGGGAAGATGGAATTCC AAGTGTAGCGGTGAAATG (SEQ ID NO: 30) | 40 | 67.6 | 0 |
| *Eggerthella* | 5B | AATCCGAGGGCTCAACCCCCGG CCG (SEQ ID NO: 31) | 25 | 67.5 | 0 |
| *Sneathia* | 7 | CTTTTAGTAGGGAAGAAAAAAT GACGGTACCTACAGAAGAAGCA ACG (SEQ ID NO: 32) | 48 | 67.1 | 0 |
| *Leptotrichia* | 8 | AGTTCACTGGACGGTAACTGAC GCTGAAGTGCGAAAG (SEQ ID NO: 33) | 37 | 67.8 | 0 |
| *M. hominis* | 12 | TAGTTGGTGAGGTAATGGCCCA CCAAGACTATGATGTTTAGC (SEQ ID NO: 34) | 42 | 67.4 | 0 |

According to another aspect of the invention the set of oligonucleotides comprises oligonucleotides specific for targeting a nucleic acid encoding 16S rRNA of one *Lactobacillus* species and oligonucleotides specific for targeting a nucleic acid encoding 16S rRNA of pathogen bacteria. Preferred combinations of an oligonucleotide targeting a nucleic acid encoding 16S rRNA of one *Lactobacillus* species and of a pathogenic bacterium are *L. crispatus*/*A. vaginae.* The set comprises oligonucloetides specific for targeting a nucleic acid encoding 16S rRNA of pathogen bacteria selected in the group comprising *Atopobium vaginae* and *Mycoplasma hominis,* the *Sneathia* group *(S. sanguinegens* and S. *amnii,* formerly *Leptotrichia amnionii),* the genera *Streptococcus, Staphylococcus, Veillonella, Megasphaera, Mobiluncus, Leptotrichia* and *Eggerthella, Bacteroides Prevotella.* The set comprises oligonucleotides specific for targeting a nucleic acid encoding 16S rRNA of *Lactobacillus* species selected in the group comprising *L. crispatus, L. iners, L. vaginalis, L. acidophilus, L. gasseri* et rel. *(L. gasseri* and *L. johnsonii*) and *L*. *jensenii* et rel. *(L. jensenii* and *L. fornicalis*).

According to another aspect, the invention relates to the use of designed probe pairs in a diagnostic method of vaginal infection and for evaluating the efficacy of the treatment of vaginal infection.

In particular, the probes targeting the bacterial species representative of vaginal microbiota of healthy and affected by vaginal infection women, have been used on DNA-microarray implemented with LDR. Said DNA-microarray is useful to identify the pathogen bacteria in vaginal fluid for the diagnosis of vaginal infection and in particular to evaluate the efficacy of treatment of vaginal infection. The vaginal infection is preferably bacterial vaginosis. The treatment of bacterial vaginosis foresees the administration of rifaximin.

The high specificity and sensitivity of the DNA microarray implemented with LDR allows to overcome the major limitations of common DNA-arrays whose discriminative power is based on hybridization.

One of the advantages of the microarray implemented with ligase detection reaction is the high specificity which is occurred in presence of perfectly matching template of three molecular entities (common probes, discriminating probes and template deriving from the PCR) avoiding the formation of aspecific bonds between similar targets.

The specificity of the microarray implemented with ligase detection reaction has been evaluated both in silico and in vitro and it has been confirmed for all set of probes since the no target template showed any interference.

The sensitivity of the microarray implemented with ligase detection reaction is about 6 ng of amplified PCR product.

The DNA- microarray can be employed with vaginal fluid without any treatment of the sample.

The microarray comprises the use of two probes and hybridized adjacently to each other deriving from 16S rRNA sequences of selected targets reported in Table 1.

The Ligase Detection Reaction (LDR) technology is based on the properties of the DNA ligation reaction, which comprises the design of a pair of two adjacent probes, as reported in Table 2A and 2B, specific for each target sequence: one oligo brings a 5'-fluorescent label, named Discriminating Probe (DS) and a second probe named Common Probe (CP), starting one base 3'-downstream of the DS and carrying a 5'-phosphate group and a unique sequence named cZipCode at its 3'-end. The probe pairs hybridize adjacently to each other on the template DNA, PCR amplicons, and the nick between the two is sealed by the ligase only if there is perfect complementarily at the junction.

Ligated fragments, obtained in presence of a perfectly matching template-DS-CP by the action of DNA ligase, are addressed to the location to a precise location onto a array, where a set of Zip-codes complementary to the cZipCodes, are arranged.

The ligated fragments carrying either the fluorescent label and a unique cZipCode sequence can be detected by laser scanning and identified according to their location on the array.

The probe pairs, DS and CP, which hybridize adjacently to each other in presence of a proper template such as a PCR amplified 16S rRNA template derived from the PCR amplification reaction on the microbial DNA encoding 16S rRNA of the selected target.

According to one aspect of the invention the nucleic acid amplification on the microbial DNA encoding 16S rRNA is carried out using universal forward primer 16S-27F(5'-AGAGTTTGATCMTGGCTCAG-3' (SEQ ID NO: 35)) and reverse primer r1492 (5'-TACGGYTACCTTGTTACGACTT-3' (SEQ ID NO: 36)), and the PCR obtained product.

Phenylen-diisothiocyanate (PDITC) activated chitosan glass slides were used as surfaces for the preparation of universal arrays, comprising a total of 49 Zip-codes. Hybridization controls (cZip 66 oligonucleotide, complementary to zip 66, 5'-Cy3-GTTACCGCTGGTGCTGCCGCCGGTA-3' (SEQ ID NO: 37)) were used to locate the submatrixes during the scanning.

The Ligase Detection Reaction (LDR) and hybridization of the products on the microarray is described in the experimental section and it was carried out with the PCR obtained products for the determination of the specificity and sensitivity of the microarray with the obtained probes.

The microarray is characterized by high sensitivity for determining the bacteria in vaginal fluid, and it is able to determinate a total bacteria amount 16S rRNA amplicon in a range from 6 to 96 ng.

According to another aspect, the invention relates to the use of microarray with the designed probes for the diagnosing vaginal bacterial infection and for evaluating the efficacy of treatment of vaginal infection.

The DNA-microarray implemented with LDR using the designed probes targeting the bacteria species selected, is employed for the diagnosis of vaginal infection and for the evaluation of the efficacy of treatment of vaginal infection.

The method is based on the detection of the presence or absence of one or more vaginal bacteria in a vaginal fluid sample comprising the step of:
a) providing vaginal fluid;
b) extracting bacterial DNA from vaginal fluid sample;
c) performing a nucleic amplification reaction on the microbial DNA encoding 16S rRNA;
d) contacting the amplified nucleic acids from step c) to common probe and discriminating probes wherein the Common Probe, CP contains a unique sequence named cZipCode at its 3'-end and the Discriminating Probe, DS brings a 5'-fluorescent label, in presence of DNA ligase;
e) subjecting the paired mix of step d) to a thermostable DNA ligase reaction in a thermal cyclic reaction with PCR amplicons as templates,
f) contacting of ligated fragments of step d) with a universal DNA Array where unique zipcode sequences have been spotted;
g) detecting the binding of one or more ligated fragments by laser scanning of the array;
h) identifying microbial pathogens in vaginal fluid samples by correlating the detected binding of the ligated fragments with the defines area of the immobilized ZipCode sequence immobilized on the array.

The microarray is useful to have information on the efficacy of antibiotic treatment of vaginal infections by the determination of the microbiota compositions.

The sensitivity of the microarray and its validation has been demonstrated analyzing vaginal samples of women affected by vaginal infection by Nugent score >3 and positive for Amsel's criteria, before the rifaximin treatment and at the end of the treatment when the women resulted recovered.

According to one aspect of the invention the method is applied for the diagnosis of vaginal infection and in particular for the evaluation of the efficacy of treatment wherein the vaginal infection is bacterial vaginosis and the antibiotic administered is rifaximin.

The evaluation of efficacy of rifaximin in the treatment of bacterial vaginosis is carried out analyzing the microbiota composition of vaginal fluid before the treatment, during the treatment and after the treatment wherein the variation of the microbiota results in a reduction of pathogen bacteria at the end of the treatment.

The rifaximin is administered at dosage of 25 mg for 5 days.

According to one aspect of the invention the method for diagnosis bacterial vaginosis and for the evaluation of the efficacy of rifaximin treatment comprises the step of:
a) providing vaginal fluid sample of woman treated with rifaximin;
b) extracting bacterial DNA from vaginal fluid;
c) performing a nucleic amplification reaction on the microbial DNA encoding 16S rRNA;
d) contacting the amplified nucleic acids from step c) to common probe and discriminating probes wherein the Common Probe, CP contains group and a unique sequence named cZipCode at its 3'-end and the Discriminating Probe, DS contains brings a 5'-fluorescent label, in presence of DNA ligase;
e) subjecting the paired mix of step d) to a thermostable DNA ligase reaction in a thermal cyclic reaction with PCR amplicons as templates,
f) contacting of ligated fragments of step d) with a universal DNA Array where unique zipcode sequences have been spotted;
g) detecting the binding of one or more ligated fragments by laser scanning of the array;
h) identifying microbial pathogens in vaginal fluid samples by correlating the detected binding of the ligated fragments with the defines area of the immobilized zip code sequence immobilized on the array.

According to one aspect of the present invention the method can be conducted with only one probe pair specific for one microorganism to detect its presence or absence. According to one aspect of the present invention can be conducted with two or more probe pairs specific for microorganisms to detect their presence or absence. According to one aspect of the present invention can be conducted with probe pairs for all microorganism according to Table 2A and 2B to detect their presence or absence.

The method of the present invention gives information about the efficacious antibiotic dosages to be chosen in order to achieve a recovery from the disease.

According to one aspect of the invention the method is applied using a set of probes designed for targeting *Lactobacillus* species and a set of probes designed for targeting pathogen bacteria, as reported in Table 1.

According to one aspect of the invention the method is applied using a pairs of probe pairs comprising one probe designed for targeting *Lactobacillus* species and the other one designed for targeting pathogen bacteria.

The probe designed for targeting pathogen bacteria is selected from the group comprising *Atopobium vaginae* and *Mycoplasma hominis,* the *Sneathia* group (S. *sanguinegens* and S. *amnii,* formerly *Leptotrichia amnionii),* the genera *Streptococcus, Staphylococcus, Veillonella, Megasphaera, Mobiluncus, Leptotrichia* and *Eggerthella, Bacteroides Prevotella.*

The probe designed for targeting *Lactobacillus* species is selected in the group comprising *L. crispatus, L. iners, L. vaginalis, L. acidophilus, L. gasseri* et rel. (*L. gasseri* and *L. johnsonii)* and *L. jensenii et* rel. (*L. jensenii and L. fornicalis*). According to another aspect of the invention the method is able to evaluate the efficacy of treatment of vaginal infection by the detection of an increase of *Lactobacillus* species which indicates an healthy and recovered vaginal eco-system, and the decrease of pathogen bacteria at the end of the treatment.

According to another aspect of the invention the method is useful for the determination of pathogen bacteria in vaginal fluid in women affected by vaginal infection.

According to another aspect of the invention the method is useful for the detection of *Lactobacillus* important to restore the healthy vaginal eco-system.

According to another aspect of the invention the method is useful for evaluating the efficacy of the treatment of vaginal infection with antibiotic at the end of the treatment.

According to another aspect of the invention the method is useful for evaluating the efficacy of the treatment of bacterial vaginosis with rifaximin during the treatment and after the treatment.

In particular the rifaximin is administered in form of vaginal tablets, wherein the rifaximin comprised in vaginal compositions can be in polymorphous, amorphous, hydrate, solvate forms and mixture thereof.

More in particular the rifaximin is administered at dosage of 25 mg for 5 days.

According to another aspect, the present invention relates to a test kit comprising a sample holding means for a vaginal sample, a diagnostic method according to the present invention and optionally the primers to perform the amplification reaction according to the present invention, the common probe and the discriminating probe. According to another aspect, the present invention relates to a kit for detecting the presence or absence of one or more vaginal bacteria in a vaginal fluid sample, comprising:
i) a microarray on which a plurality of specific recognition elements containing one or more unique Zipcode sequences have been immobilized in discrete measurement areas of known location,
ii) a sample holding means for a vaginal sample,
iii) a discriminating probe comprising a 5'-fluorophore-modified oligonucleotide specific for a vaginal bacterium or bacteria group and being selected from the sequences of Table 2A and B,
iv) a common probe specific for a vaginal bacterium or bacteria group, starting one base 3'-downstream of the discriminating probe and comprising a 5'-phosphate group and a unique sequence named cZipcode at its 3'-end and being selected from the sequences of Table 2A and 2B, and
v) a ligation mix.

According to another aspect, the present invention relates to the use of a kit or a set of oligonucleotides for the specific quantitative and/or qualitative determination of a 16S rRNA gene or fragment thereof derived from a vaginal bacterium.

According to another aspect, the present invention relates to the use of kit for evaluating the efficacy of an antibiotic treatment during the treatment and the relapse after the end of the treatment of a vaginal infection.

According to another aspect, the present invention relates to the use of kit for determining an efficacious antibiotic dosage in the treatment of a vaginal infection. According to one aspect of the invention the method is applied using a set of probes designed for targeting *Lactobacillus* species and a set of probes designed for targeting pathogen bacteria, as reported in Table 1.

According to another aspect of the present invention the kit comprise a pairs of probes comprising one probe designed for targeting *Lactobacillus* species and the other one designed for targeting pathogen bacteria.

The probe designed for targeting pathogen bacteria is selected from the group comprising *Atopobium vaginae* and *Mycoplasma hominis,* the *Sneathia* group (S. *sanguinegens* and S. *amnii,* formerly *Leptotrichia amnionii),* the genera *Streptococcus, Staphylococcus, Veillonella, Megasphaera, Mobiluncus, Leptotrichia* and *Eggerthella, Bacteroides Prevotella.*

The probe designed for targeting *Lactobacillus* species is selected in the group comprising *L. crispatus, L. iners, L. vaginalis, L. acidophilus, L. gasseri* et rel. (*L*. *gasseri* and *L. johnsonii)* and *L. jensenii et* rel. (*L. jensenii and L. fornicalis*). According to another aspect, the present invention relates to the use of the diagnostic method or a test kit according to the present invention for evaluating efficacy of the treatment of vaginal infection with antibiotics. Preferably the vaginal infection is bacterial vaginosis and the antibiotic administered is rifaximin.

In another aspect, the present invention relates to the use of the microarray or a test kit at the end of the rifaximin treatment of vaginal infection in order to evaluate the efficacy of the treatment of vaginal infections and to estimate if the patients could relapse.

The present invention is further illustrated by the following figures and examples without being restricted thereto.

### Description of the figures of the invention

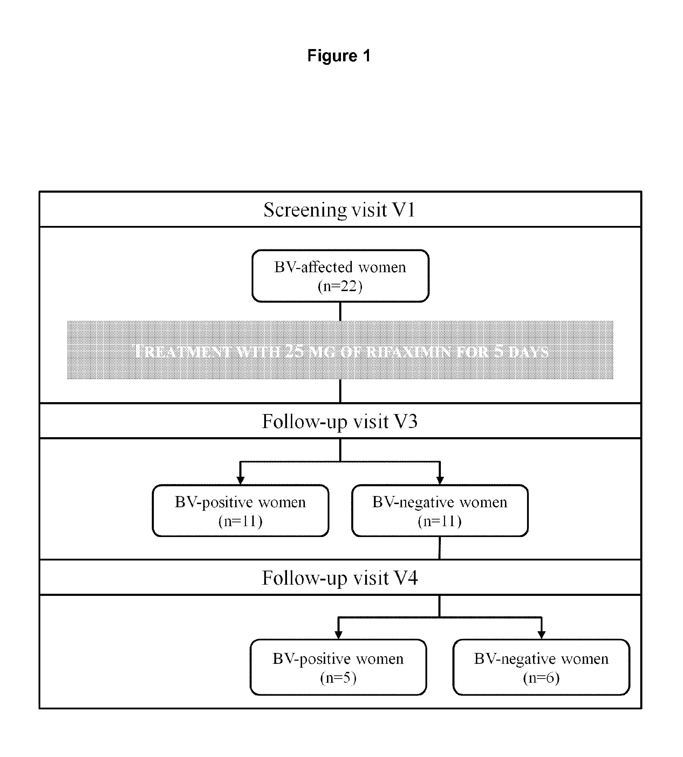

FIG. 1: Design of the clinical study reported in WO 2013/017928 used as validation model of the method object of the present invention.

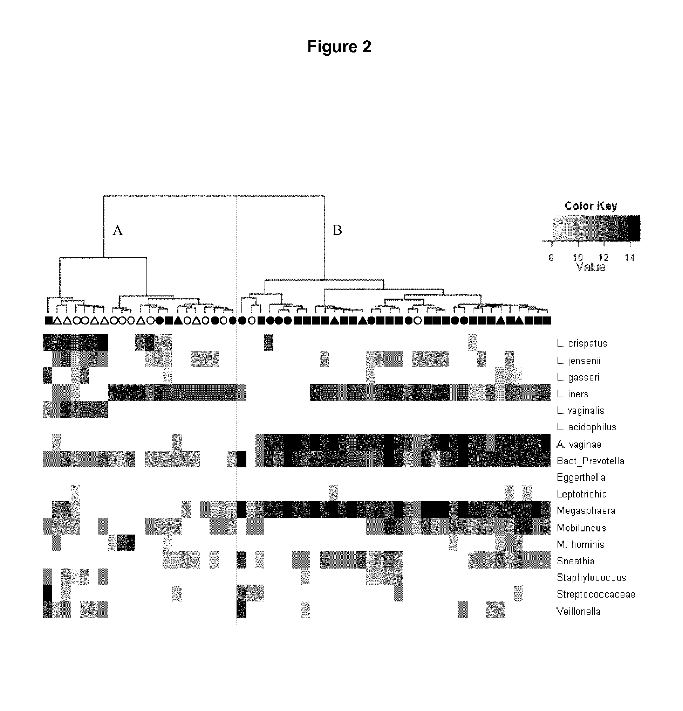

FIG. 2 shows hierarchical clustering and heat map of the microarray data related to the analyzed samples. The dotted vertical line separates the two clusters A and B. The open and filled symbols represent BV-negative and BV-positive samples, respectively. The squares, circles and triangles represent samples collected at visits V1, V3 and V4, respectively. In a scale of grey the mean log intensity of fluorescence values for each bacterial groups are plotted. Ward's clustering method was used.

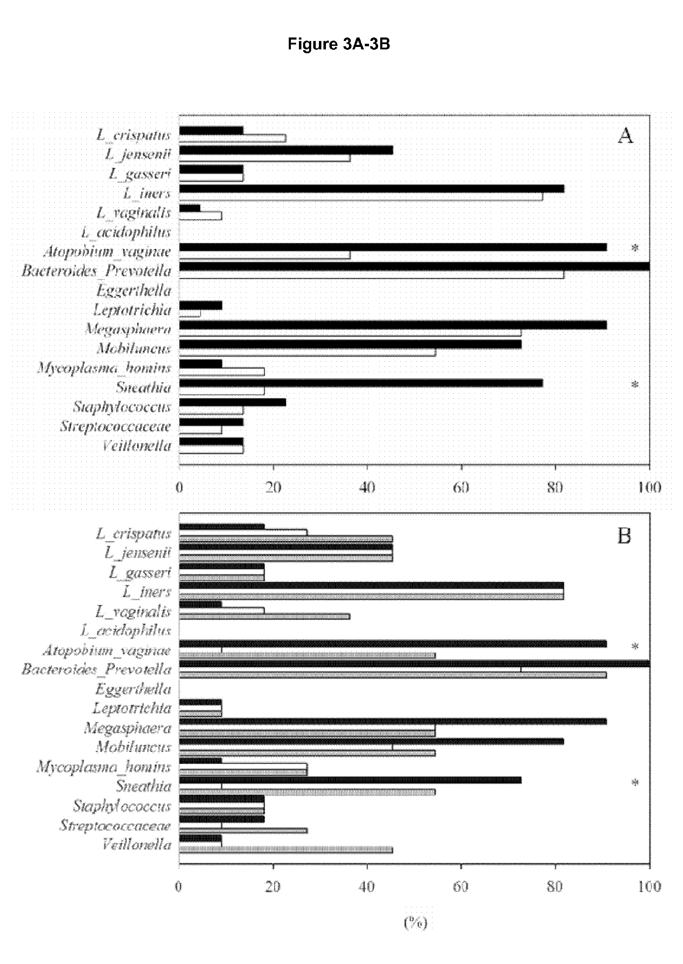

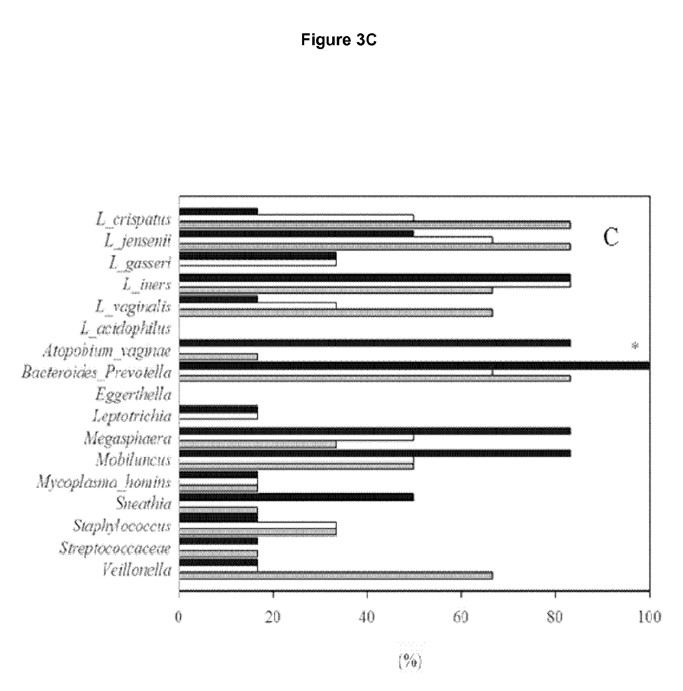

FIG. 3A, 3B and 3C show prevalence rates of bacterial groups in relation to the different time points of the study. 3A) Prevalence at V1 and V3 for all the women enrolled in the study (n=22). 3B) Prevalence at enrolled visit V1, V3 and V4 for the subset of women who went in remission at V3 (n=11). 3C) Prevalence at V1, V3 and V4 for the subset of women who maintained remission at V4 (n=6). Black, white and grey bars indicate prevalence at V1, V3 and V4, respectively. Differences in the prevalence rates of each bacterial group were tested using Chi Square analysis of contingency, stars indicate P<0.05.

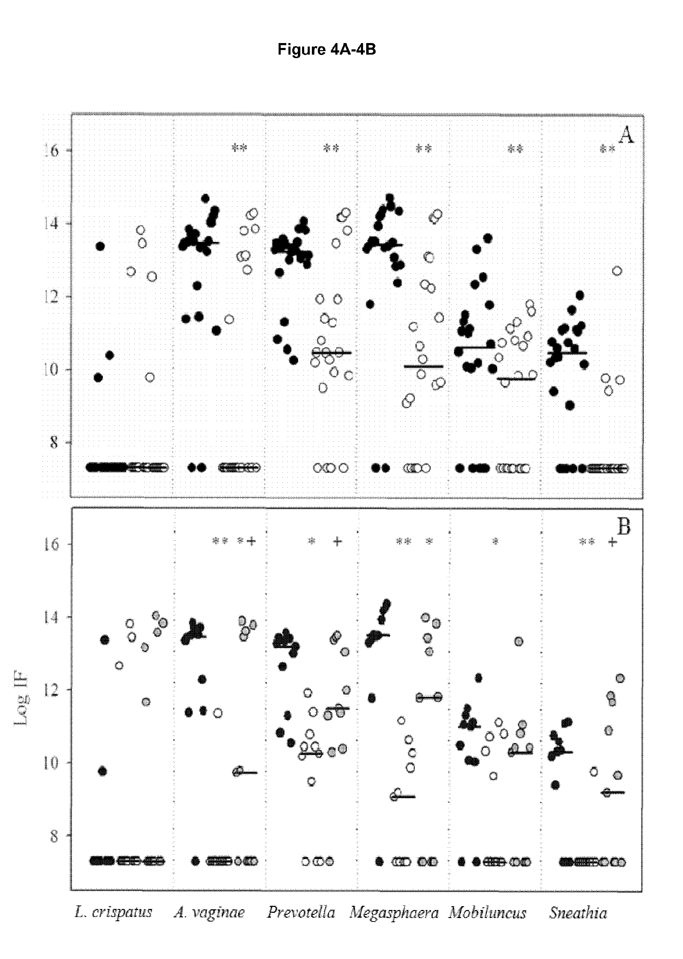

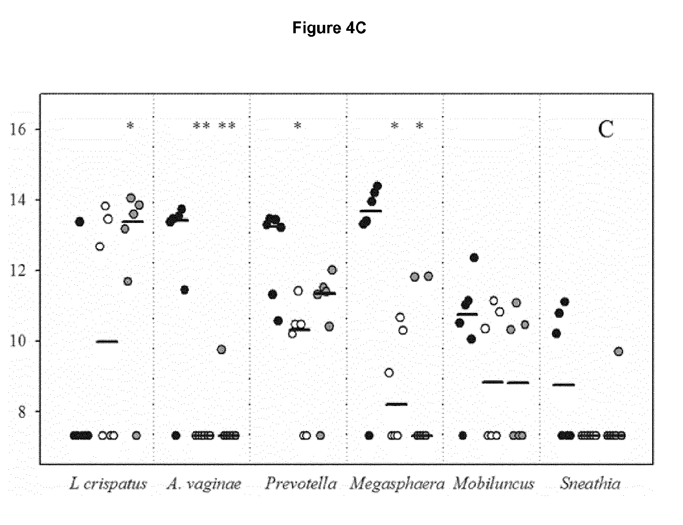

FIG. 4A, 4B and 4C show scatter plots of log IF values of the target bacteria at the different time points of the study. The plots represent data for *L. crispatus, A. vaginae, Prevotella, Megasphaera, Mobiluncus* and *Sneathia* which significantly changed over the study. 4A) Scatter plots of log IF related to V1 and V3 for all the women enrolled in the study (n=22). 4B) Scatter plots of log IF related to V1, V3 and V4 for the subset of women who went in remission at V3 (n=11). 4C) Scatter plots of log IF related to V1, V3 and V4 for the subset of women who maintained remission at V4 (n=6). The scatter plots show the distributions of relative quantification data, with the horizontal bars representing the median values. Black, white and grey circles indicate data relative to V1, V3 and V4, respectively. Differences in the log IF values corresponding to each bacterial group at the different time points of the study were analyzed using Wilcoxon's signed rank test. Single star indicates P<0.05 respect to enrolled visit; double star indicates P<0.005 respect to V; cross indicates P<0.05 respect to V1.

### EXAMPLES

### Example 1: Sample preparation

22 European pre-menopausal, non -pregnant women were selected to test the diagnostic method. At the screening visit (V1) the women were diagnosed with BV as they presented Nugent score >3 and positive for > 3 Amsel's criteria. The patient received a daily 25 mg rifaximin tablet according to US13/559,013 for 5 days, administered intra-vaginally at bed time.

Diagnosis of remission was made after 7 days after the end of the treatment (V3) for the women who presented Nugent score < 3 and were positive for < 2 Amsel's criteria. Patients showing remission at V3 (11) attended a second follow-up visit (V4) 28 days after the end of the treatment. Standardised vaginal rinsing with 2 ml of saline were collected for molecular studies at Vi, V3 and V4 by flushing and re-aspirating the fluid through a 22 Gauge needle in the left, central and right upper vaginal visual. The vaginal samples were stored at -80°C until use.

### Example 2: DNA preparation

The bacterial DNA from *Atopobium vaginae* DSM15829, *Eggerthella lenta* DSM2243, *Lactobacillus crispatus* DSM20584, *L. iners* DSM13335, *L. jensenii* DSM20557, *L*. *vaginalis* DSM5837, *Leptotrichia buccalis* DSM1135, *Megasphaera elsdenii* DSM20460, *Mobiluncus curtisii* DSM2711, *Mycoplasma hominis* DSM19104, *Prevotella bivia* DSM20514, *Sneathia amnii* DSM16630, S. *sanguinegens* DSM22970, *Streptococcus agalactiae* DSM2134, *Veillonella parvula* DSM2008 was directly obtained from the DSMZ (Braunschweig, Germany).

Genomic DNA from *Lactobacillus acidophilus* DSM20079, *L. gasseri* DSM20243 and *Staphylococcus aureus* ATCC12600 was extracted from 109 bacterial cells by using the DNeasy Blood and Tissue Kit 50 (Qiagen, Düsseldorf, Germany) following the manufacturer instructions. *Lactobacillus* strains were grown on De Man-Rogosa-Sharpe (MRS) broth with cysteine (0.5 g/L) at 37°C, under anaerobic atmosphere (Anaerocult, Merck, Darmstadt, Germany). S. *aureus* ATCC12600 were grown at 37°C aerobically on Luria-Bertani (LB) broth.

Total bacterial DNA was also extracted from vaginal rinsings of 55 samples of the 22 Eureopean pre-menopausal non pregnant women of example 1. DNA amount were quantified using NanoDrop ND-1000 (NanoDrop® Technologies, Wilmington, DE).

### Example 3: Polymerase Chain Reaction (PCR)

16S rRNA gene was amplified using the following universal primer set: 27F (5'-AGAGTTTGATCMTGGCTCAG-3' (SEQ ID NO: 35)) and reverse primer 1492R (5'-TACGGYTACCTTGTTACGACTT-3' (SEQ ID NO: 36)). The reaction mixtures included 500 nM concentrations of each primer, 200 µM concentrations of each deoxynucleoside triphosphate, 2 mM MgCl2, 2.5 U of Gotaq Flexy polymerase (Promega, Madison, WI), and 50 ng of genomic DNA in a final volume of 50 µl. Prior to amplification, the DNA was denatured for 5 min at 95°C. Amplification comprised of 40 cycles at 95°C for 60 s, 60°C for 30 s, and 72°C for 90 s. After the cycles, an extension step (5 min at 72°C) was performed.

PCR products were purified with a High Pure PCR Clean up Micro kit and the purified amplicons were quantified by using the NanoDrop ND-1000.

### Example 4: Oligonucleotide probe design

16S rRNA sequences of selected bacterial groups (single species, related species, or genera belonging to the human vaginal microbiota) of particular interest for the vaginal microbial ecosystem were rationally selected and a local database was created downloading the full-length sequences available in the Ribosomal Database Project (RDP) repository, generating: a) for each of the desired targets, a so-called "positive set", comprising all the sequences the oligonucleotide probes should ideally detect, and ,b) a "negative set" database including all the sequences of the species the oligonucleotide probes must not detect.

Positive and negative set sequences were multiply-aligned in ClustalW and group specific consensus sequences were extracted, with a cut-off of 75% identity for base calling. Nucleotides which occurred at lower frequencies were replaced by the appropriate IUPAC ambiguity code.. For each target, its consensus sequence was searched in order to identify positions capable to discriminate between target and non-target sequences (i.e.: those belonging to the negative set) and DS and CP oligonucleotides were designed. DS brought the discriminating position at its 3' end, whereas the CP sequence started 1-base 3' of the discriminating position; probe length was appropriately selected in order to have all the probes with a homogeneous melting temperature around 67 °C. The selection for the best probe pair was made requiring each oligonucleotide to: a) have less than 4 ambiguous bases; b) be specific for all (or the vast majority of) the positive set of each target; c) be non-specific for any (or few) non-target species or group. The designed probes pairs had an average melting temperature (Tm) of 67.6±1.5 °C and a average length of 37.1 ±14.9 nucleotides.

The list of probes, Discriminating probe and Common Probe is reported in Tables 2A and B.

### Example 5: Probes Specificity

The specificity of the designed LDR probe pairs was tested by using 16S rRNA PCR amplicons from 16 microorganisms members of the human vaginal microbiota. Amplicons were prepared by amplification of genomic DNA provided by DSMZ or extracted from pure cultures. All the 16S rRNA amplicons were properly recognized in separate LDR hybridization reactions with the entire probe set of the array. For each of the 16S rRNA template only group-specific spots, and spots corresponding to the hybridization controls showed positive signals (P<0.0005).

### Example 6: Sensitivity of the microarray

The detection limits of the diagnostic method, were assessed carrying out LDR-UA experiments with different concentrations of an artificial mix containing equal amounts of 16S rRNA amplicons from 6 members of the human vaginal microbiota. The 16S rRNA amplicons from *L. acidophilus, L. crispatus, L. jensenii, M. hominis, P. bivia* and *Streptococcus* were all specifically recognized in a range of total DNA amount from 6 to 96 ng (P<0.0005), demonstrating the high sensitivity and specificity.

### Example 7: Ligation detection reaction (LDR) combined with a universal array (UA) approach

The LDR was carried out in a final volume of 20 µl containing 20 mM Tris-HCl (pH 7.5), 20 mM KCI, 10 mM MgCl2, 0.1% NP-40, 0.01 mM ATP, 1 mM dithiothreitol, 250 fmol of each oligo per each probe pair, 250 fmol of synthetic template (5'-AGCCGCGAACACCACGATCGACCGGCGCGCGCAGCTGCAGCTTGCTCATG-3'), 10 fmol of the hybridization control, and with different quantities of purified PCR products: a) all LDRs for specificity tests were performed on 10 ng of initial PCR product; b) sensitivity tests were performed with decreasing PCR product amount from 96 ng to 6 ng; c) LDR experiments on human vaginal samples were performed on 48 ng of PCR product.

After the reaction mixture was preheated for 2 min at 94°C and centrifuged for 1 min, 4U of Pfu DNA ligase was added. The LDR was cycled for 30 rounds at 90°C for 30 s and at 60°C for 4 min in a GeneAmp PCR system 9700 thermal cycler.

### Example 8: Array hybridization

The hybridization mixture had a total volume of 65 µl and contained 20 µl of LDR mixture, 5x SSC, and 0.1 mg of salmon sperm DNA/ml. Hybridization was carried out in a chamber in the dark at 65°C for 1 h in a temperature-controlled water bath. After hybridization, the slide was washed at 65°C for 15 min in pre-warmed 1 × SSC and 0.1% sodium dodecyl sulfate.

### Example 9: Signal detection and data analysis

All arrays were scanned with ScanArray 5000 scanner (Perkin Elmer Life Sciences, Boston, MA, USA), at 10 µm resolution and the fluorescence intensity (IF) was quantitated by ScanArray Express 3.0 software. In order to be able to compare data from different samples and acquisitions, a normalization procedure based on the IFs of the synthetic ligation control signal was applied as follows: (a) outlier values (2.5-fold above or below the average) were discarded; (b) a correction factor was calculated in order to set the average IF of a ligation control to 50000 (n=6); (c) the correction factor was applied to both the probes and background IF values. Significantly present probe pairs were determined using a one-sided t-test comparing, for each ZipCode, the distribution of IFs along all replicates with the distribution of IFs of negative controls (i.e.: "Blanks", where only printing buffer has been spotted).

### Example 10: In silico hybridization matrix

In silico checks versus the publicly available database RDP (using the tool Probe Match) were, then, performed for assessing probe pair specificity. All the selected probe pairs were required to perfectly match the sequences of the positive set and to possess at least one mismatch at the 3' end of the discriminating probe respect to the entire negative set.

### Example 11: Use of microarray on the effect of rifaximin in women affected by BV

Microarray was validated on the samples collected by women affected by vaginal infection as described in Example1 and prepared according to Example 9.

The IF signals registered for each probe in relation to the different time of sample collection (V1, V3 and V4) and clinical diagnosis (P = BV-positive, N = BV-negative, U= unknown) were compared. Hierarchical clustering based on log IF data of the target bacterial groups in a heat map showed that BV-positive and BV-negative samples tended to group separately (P <0.001), regardless of the collection time point as reported in Figure 2. Almost all BV-positive samples were characterized by higher abundance (P < 0.04) of *A. vaginae, Bacteroides*/*Prevotella, Megasphaera, Mobiluncus* and *Sneathia* compared to BV-negative samples. In most of BV-positive vaginal fluids L. iners was the only *Lactobacillus* spp. to be present (52%). *L. jensenii* was also present in 10 samples (32%), while lactobacilli were absent in 5 samples (16%). In BV-negative and V4 samples, *L. iners* was, again, the only or the most abundant *Lactobacillus* spp. (60%). A small subgroup, principally composed by samples collected at V4, was characterized by the presence of *L. crispatus, L. jensenii* and *L. vaginalis,* which are known to be markers of health.

Figure 3 represents the prevalence rates of each analyzed bacterial group in relation to the different time points of the study. The probes specific for *L. acidophilus* species and *Eggerthella* genus did not give significant signal in any of the analyzed samples (prevalence = 0%). This may be due to the low concentration of the target bacteria in the vaginal ecosystem, or to a primer bias that leads to under-representation of these specific bacterial groups in the complex amplified samples, as in the case of G. *vaginalis.* The analysis of the results regarding V1 and V3 samples collected from the totality of the women enrolled in the study showed a significant drop (P < 0.001) in the number of women harbouring *A. vaginae* (V1: 90.9%, V3: 36.4%) and *Sneathia* ssp. (V1: 77.3%, V3: 18.2%) after antibiotic intake. Among the other infection-related bacteria, a slight reduction in prevalence was also observed for *Bacteroides*/*Prevotella* spp. (V1: 100%, V3: 81.8%), *Megasphaera* spp. (V1: 90.9%, V3: 72.7%), *Mobiluncus* spp. (V1: 71.7%, V3:54.6%). A trend toward the increase of prevalence at V3 was registered for *M. hominis* (V1: 9.1%, V3: 18.2%), which is known to be resistant to rifaximin, while no change involved *Veillonella* spp. For what concerns the lactobacilli population, the *Lactobacillus* spp. distribution remained almost unchanged at V3. The higher prevalence rates at V1 was observed for *L*. *iners* (81.8%) followed by *L. jensenii* (45.5%), which slightly decreased at V3 (77.3% and 36.4%, respectively). Figure 3B shows the prevalence of the vaginal communities at V1, V3 and V4 in the subset of women who went in remission at V3. The bacterial groups whose prevalence rates was reduced at V3 showed an increased occurrence at V4, albeit maintaining lower values compared to the baseline. In particular, *A. vaginae* [V1: 90.9%, V3: 9.1%, V4: 54.6% (P < 0.001)] and *Sneathia* [V1: 72.7%, V3: 9.1%, V4: 54.6% (P = 0.009)] showed the highest susceptibility to the antibiotic activity as demonstrated by the low prevalence at V3 and the partial maintenance of the effect at V4. Also *Megasphaera* (V1: 90.9%, V3: 54.6%, V4: 54.6%) and *Mobiluncus* (V1: 81.8%, V3: 45.5%, V4: 54.6%) showed a trend toward the reduction in prevalence after rifaximin treatment. On the contrary, *M*. *hominis* (V1: 9.1%, V3: 27.3%, V4: 27.3%), and *Veillonella* spp. (V1: 9.1%, V3: 9.1%, V4: 45.5%) slightly increased their percentage at V4, showing a low susceptibility to the antibiotic. At V1, *L. iners* and *L. jensenii* showed the highest prevalence rates (81.8% and 45.5%, respectively), which were maintained throughout the study. Noteworthy, *L. crispatus* (V1: 18.2%, V3: 27.3%, V4: 45.5%) and *L. vaginalis* (V1: 9.1%, V3: 18.2%, V4: 36.4%) showed a trend to increase their prevalence rates in the study period. These results suggest that Lactobacillus spp., especially *L*. *crispatus* and *L. vaginalis,* are not affected by rifaximin and what's more they raise their presence after one month from the end of the therapy, probably taking advantage of the disappearance of other BV-related species. In order to further assess the impact of rifaximin on the composition of the vaginal microbiota, the distributions of log IF values registered at the different time points of the study for the target bacteria were plotted and it is reported in Figure 4. The scatter plots represent data relative to *A. vaginae, Prevotella, Megasphaera, Mobiluncus* and *Sneathia* which significantly changed over the study. The analysis of the variations that occurred in all the women enrolled in the study revealed a remarkable droop (P < 0.001) in the log IF values after rifaximin treatment. When considering the subset of women in remission at V3, an increase of the signal was registered for *A. vaginae, Prevotella* and *Sneathia* at V4, with respect to V3 as reported in Figure 3 B. The pathogen bacteria *A. vaginae* and *Megasphaera* log IF values at V4 were still significantly lower than those at V1. The efficacy of rifaximin on the composition of the vaginal microbiota, the distributions of log IF values registered at the different time points of the study for the target bacteria is reported in Figure 4. The scatter plots represent data relative to *L. crispatus, A. vaginae, Prevotella, Megasphaera, Mobiluncus* and *Sneathia* which significantly changed over the study. The analysis of the variations that occurred in all the women enrolled in the study, Figure 4A, revealed a remarkable droop (P<0.001) in the median log IF values of the considered bacteria after rifaximin treatment, with the exception of *L. crispatus* that showed no variation. When considering the subset of women who went in remission at V3,

Figure 4B, an increase of the signal was registered for *A. vaginae, Prevotella* and *Sneathia* at V4 (P<0.05), with respect to V1.

*A. vaginae* and *Megasphaera* median log IF values at V4 were still significantly lower than those at V1 (P<0.02); *L. crispatus* median log IF did not changed in this group of patients. Interestingly, women who maintained remission at V4, Figure 5C, shows a significant increase of *L. crispatus* signal at V4 with respect to V1 (P=0.02). *A. vaginae* and *Megasphaera* significantly decreased at V3 and V4 (P<0.01), while *Prevotella* showed a reduction at V3 (P=0.02) followed by a slight increase at V4. *Mobiluncus* and *Sneathia* did not show significant changes, even if the signal tended to decrease at V3 with a maintenance of the effect at V4.

These results, together with the previously described prevalence data, suggest that microarray is able to demonstrate the efficacy of the treatment with 25 mg of rifaximin once daily for 5 days in the reduction of the pathogen bacteria without compromising the population of lactobacilli, as found in WO 2013/017928.

The microarray analysis has demonstrated an increase of *L. crispatus* signal in the subset of women who maintained remission after one month of therapy.

### Conclusion remarks

The method object of the present invention for the identification of vaginal pathogens is the first molecular diagnostic tool able to identify a wide range of clinically relevant bacteria and yeast directly from vaginal fluid in an appreciate period of time. The combination of PCR amplification with the microarray hybridization presents a powerful tool for pathogen identification. It excels common technologies in speed (about 8 hours) while performing at an extremely high specificity. Analysis of 16S rRNA gene has been reported before to allow a more robust reproducible and accurate testing that classical culture-based detection methods.

Sensitivity was increased by the use of a pair of two adjacent probes. The accurate selection of the probe sequences, associated to a ligation-mediated reaction gave high specificity for diagnosing bacterial infection and for diagnosing the efficacy of the antibiotic treatment in particular rifaximin treatment.

## Claims

1. A method of detecting the presence or absence of one or more vaginal bacteria in a vaginal fluid sample comprising the steps of
a) providing a vaginal fluid sample,
b) extracting the bacterial DNA from the bacteria contained in the sample,
c) performing a nucleic acid amplification reaction on the microbial DNA encoding 16S rRNA,
d) contacting the amplified nucleic acids from step c) with a pair of two adjacent probes specific for each bacterial species, a discriminating probe comprising a 5'-fluorophore-modified oligonucleotide, and a common probe starting one base 3'-downstream of the discriminating probe and comprising a 5'-phosphate group and a unique sequence named cZipcode at its 3'-end, to obtain a paired mix of the two probes,
e) subjecting the paired mix of step d) to a thermostable DNA ligase reaction in a thermal cyclic reaction with PCR amplicons as templates,
f) contacting the ligated fragments of step e) with a universal DNA array on which the unique Zipcode sequences have been spotted,
g) detecting the binding of one or more ligated fragments by laser scanning of the array,
h) identifying the presence or absence of one or more vaginal bacteria by correlating the detected binding of the ligated fragments with the defined area of the unique cZipcode sequence immobilized on the array by laser scanning,
wherein a set of probes are used comprising
- a probe pairs designed for targeting a nucleic acid encoding 16S rRNA of a *Lactobacillus* species selected in the group comprising *L. crispatus, L. iners, L. vaginalis, L. acidophilus, L. gasseri* et rel. *(L. gasseri* and *L. johnsonii)* and *L. jensenii* et rel. *(L. jensenii* and *L. fornicalis*); and
- a probe pairs designed for targeting a nucleic acid encoding 16S rRNA of a pathogen bacteria selected from the group comprising *Atopobium vaginae* and *Mycoplasma hominis,* the *Sneathia* group *(S. sanguinegens* and S. *amnii,* formerly *Leptotrichia amnionii),* the genera *Streptococcus, Staphylococcus, Veillonella, Megasphaera, Mobiluncus, Leptotrichia* and *Eggerthella, Bacteroides Prevotella.*

2. The method according to claim 1, **characterized in that** the nucleic acid amplification reaction is performed by a PCR reaction.

3. The method according to claim 1 or 2, **characterized in that** for each bacterial species the common probe is selected from SEQ ID NOs: 1 to 17 and the discriminating probe is selected from SEQ ID NOs: 18 to 34.

4. A kit for detecting the presence or absence of one or more vaginal bacteria in a vaginal fluid sample, comprising
i) a microarray on which a plurality of specific recognition elements containing one or more unique Zipcode sequences have been immobilized in discrete measurement areas of known location,
ii) a sample holding means for a vaginal sample,
iii) a discriminating probe comprising a 5'-fluorophore-modified oligonucleotide specific for a vaginal bacterium and being designed for targeting 16S rRNA of *Lactobacillus* species selected in the group comprising *L. crispatus, L. iners, L. vaginalis, L. acidophilus, L. gasseri* et rel. *(L. gasseri* and *L. johnsonii)* and *L. jensenii* et rel. *(L. jensenii* and *L. fornicalis*) or of pathogen bacteria selected from the group comprising *Atopobium vaginae* and *Mycoplasma hominis,* the *Sneathia* group (*S*. *sanguinegens* and S. *amnii,* formerly *Leptotrichia amnionii*)*,* the genera *Streptococcus, Staphylococcus, Veillonella, Megasphaera, Mobiluncus, Leptotrichia* and *Eggerthella, Bacteroides Prevotella,*
iv) a common probe specific for a vaginal bacterium, starting one base 3'-downstream of the discriminating probe and comprising a 5'-phosphate group and a unique sequence named cZipcode at its 3'-end and being designed for targeting 16S rRNA of *Lactobacillus* species selected in the group comprising L. *crispatus, L. iners, L. vaginalis, L. acidophilus, L. gasseri* et rel. (*L. gasseri* and *L. johnsonii*) and *L*. *jensenii* et rel. *(L. jensenii* and *L. fornicalis*) or of pathogen bacteria selected from the group comprising *Atopobium vaginae* and *Mycoplasma hominis,* the *Sneathia* group *(S. sanguinegens* and *S*. *amnii,* formerly *Leptotrichia amnionii),* the genera *Streptococcus, Staphylococcus, Veillonella, Megasphaera, Mobiluncus, Leptotrichia* and *Eggerthella, Bacteroides Prevotella,* and
v) a ligation mix.

5. The kit according to claim 4, **characterized in that** it comprises pairs of probes, wherein the discriminating probe is selected from SEQ ID NOs: 1 to 16 and the common probe is selected from SEQ ID NOs: 17 to 32.

6. A set of oligonucleotides specific for targeting a nucleic acid encoding 16S rRNA of a vaginal bacterium, **characterized in that** the set comprises
- oligonucleotides specific for targeting a nucleic acid encoding 16S rRNA of Lactobacillus species selected in the group comprising *L. crispatus, L. iners, L. vaginalis, L. acidophilus, L. gasseri* et rel. *(L. gasseri* and *L. johnsonii)* and *L. jensenii* et rel. *(L. jensenii* and *L. fornicalis*); and
- oligonucleotides specific for targeting a nucleic acid encoding 16S rRNA of pathogen bacteria selected in the group comprising *Atopobium vaginae* and *Mycoplasma hominis,* the *Sneathia* group *(S. sanguinegens* and S. *amnii,* formerly *Leptotrichia amnionii),* the genera *Streptococcus, Staphylococcus, Veillonella, Megasphaera, Mobiluncus, Leptotrichia* and *Eggerthella, Bacteroides Prevotella.*

7. The set of oligonucleotides according to claim 6, **characterized in that** the set comprises a first probe selected from SEQ ID NOs: 1 to 17 and a second probe selected from SEQ ID NOs: 18 to 32, whereby the first and the second probe are specific for the same organism.

8. Use of a kit according to claims 4 or 5 or a set of oligonucleotides according to claims 6 or 7 for the specific quantitative and/or qualitative determination of a 16S rRNA gene or fragment thereof derived from a vaginal bacterium.

9. The use of claim 8 for diagnosing a vaginal infection.

10. The use of claim 8 for evaluating the efficacy of an antibiotic treatment during the treatment and the relapse after the end of the treatment of a vaginal infection.

11. The use of claim 8 for determining an efficacious antibiotic dosage in the treatment of a vaginal infection.


SEQUENCE LISTING

<110> Alfa Wassermann S.p.A.

<120> Identification of vaginal bacteria

<130> S-123

<160> 34

<170> BiSSAP 1.3

<210> 1
<211> 34
<212> DNA
<213> Artificial Sequence

<220>
<223> Synthetiyc primer

<400> 1
gcccaaagtc ggtggcctaa ccatttggag ggaa                                    34

<210> 2
<211> 44
<212> DNA
<213> Artificial Sequence

<220>
<223> Synthetic primer

<400> 2
cctggaaaca gatgctaata ccggataaca acagatgatg ccta                         44

<210> 3
<211> 45
<212> DNA
<213> Artificial Sequence

<220>
<223> Synthetic primer

<400> 3
gaaccttacc aggtcttgac atctagtgcc atttgtagag ataca                        45

<210> 4

<211> 44
<212> DNA
<213> Artificial Sequence

<220>
<223> Synthetic primer

<400> 4
gcaacccttg ttaatagttg ccagcattaa gttgggcact ctat 44

<210> 5
<211> 52
<212> DNA
<213> Artificial Sequence

<220>
<223> Synthetic primer

<400> 5
ggtagtgaag aaagatagag gtagtaactg gcctttattt gacggtaatt ac 52

<210> 6
<211> 43
<212> DNA
<213> Artificial Sequence

<220>
<223> Synthetic primer

<400> 6
ggtgctaata ccggataaga aagcagatcg catgatcagc tta 43

<210> 7
<211> 38
<212> DNA
<213> Artificial Sequence

<220>
<223> Synthetic primer

<400> 7
cgtaaagcga gcgcaggcgg ttwdataagt ctgaagtt 38

<210> 8
<211> 46
<212> DNA

<213> Artificial Sequence

<220>
<223> Synthetic primer

<400> 8
caacccttaa gcttagttgc cakcattaag ttgggcactc targtt                46

<210> 9
<211> 28
<212> DNA
<213> Artificial Sequence

<220>
<223> Synthetic primer

<400> 9
caccgcccgt cacaccacga aagtcgga                                    28

<210> 10
<211> 39
<212> DNA
<213> Artificial Sequence

<220>
<223> Synthetic primer

<400> 10
ctacaatggc tcttaataga gggaagcgaa ggagcgatc                       39

<210> 11
<211> 33
<212> DNA
<213> Artificial Sequence

<220>
<223> Synthetic primer

<400> 11
cattaagatc cacctgggga gtacgccggc aac                              33

<210> 12
<211> 34
<212> DNA
<213> Artificial Sequence

<220>
<223> Synthetic primer

<400> 12
cgtcgcgtct gtcgtgaaag ccagcagctt aact                                34

<210> 13
<211> 28
<212> DNA
<213> Artificial Sequence

<220>
<223> Synthetic primes

<400> 13
ccgctcctga taccggcagg cttgagtc                                       28

<210> 14
<211> 28
<212> DNA
<213> Artificial Sequence

<220>
<223> Synthetic primer

<400> 14
cgtaggcggc ctctcaagcg ggatctct                                       28

<210> 15
<211> 48
<212> DNA
<213> Artificial Sequence

<220>
<223> Synthetic primer

<400> 15
gatccagcaa ttctgtgtgt gtgaagaagg ttttaggact gtaaaaca                 48

<210> 16
<211> 45
<212> DNA
<213> Artificial Sequence

<220>
<223> Synthetic primer

<400> 16
gaggtgaaat tcgtagatat gtgcaggaat gccgatgatg aagat                45

<210> 17
<211> 39
<212> DNA
<213> Artificial Sequence

<220>
<223> Synthetic primer

<400> 17
gtaaggcgcc actaaaagat gagggtgcgg aacattagt                39

<210> 18
<211> 27
<212> DNA
<213> Artificial Sequence

<220>
<223> Synthetic primer

<400> 18
ccgcctaagg cagggcagat gactggg                27

<210> 19
<211> 46
<212> DNA
<213> Artificial Sequence

<220>
<223> Synthetic primer

<400> 19
tcaactgttt aaaagatggt tctgctatca ctcttggatg gacctg                46

<210> 20
<211> 33
<212> DNA
<213> Artificial Sequence

<220>
<223> Synthetic primer

<400> 20

aagttccctt cggggacgct aagacaggtg gtg 33

<210> 21
<211> 30
<212> DNA
<213> Artificial Sequence

<220>
<223> Synthetic primer

<400> 21
tgagactgcc ggtgacaaac cggaggaagg 30

<210> 22
<211> 35
<212> DNA
<213> Artificial Sequence

<220>
<223> Synthetic primer

<400> 22
ttagaaagtc acggctaact acgtgccagc agccg 35

<210> 23
<211> 34
<212> DNA
<213> Artificial Sequence

<220>
<223> Synthetic primer

<400> 23
taaaaggcgg cgtaagctgt cgctatggga tggc 34

<210> 24
<211> 47
<212> DNA
<213> Artificial Sequence

<220>
<223> Synthetic primer

<400> 24
aaaggcwgtg gctyaaccat wgtacgcttt ggaaactgtt aaacttg 47

<210> 25
<211> 29
<212> DNA
<213> Artificial Sequence

<220>
<223> Synthetic primer

<400> 25
gactgccggt gacaaaccgg aggaaggtg                                          29

<210> 26
<211> 29
<212> DNA
<213> Artificial Sequence

<220>
<223> Synthetic primer

<400> 26
agtgcccaaa gccggtgggg taaccttcg                                          29

<210> 27
<211> 34
<212> DNA
<213> Artificial Sequence

<220>
<223> Synthetic primer

<400> 27
cggagcaaac cccaaaaaca gagtcccagt tcgg                                    34

<210> 28
<211> 32
<212> DNA
<213> Artificial Sequence

<220>
<223> Synthetic primer

<400> 28
ggtgaaactc aaaggaattg acgggggccc gc                                      32

<210> 29

<211> 25
<212> DNA
<213> Artificial Sequence

<220>
<223> Synthetic primer

<400> 29
gttggtctgc ggtgggtacg ggcgg                                          25


<210> 30
<211> 40
<212> DNA
<213> Artificial Sequence

<220>
<223> Synthetic primer

<400> 30
tggtagggga agatggaatt ccaagtgtag cggtgaaatg                          40


<210> 31
<211> 25
<212> DNA
<213> Artificial Sequence

<220>
<223> Synthetic primer

<400> 31
aatccgaggg ctcaaccccc ggccg                                          25


<210> 32
<211> 48
<212> DNA
<213> Artificial Sequence

<220>
<223> Synthetic primer

<400> 32
cttttagtag ggaagaaaaa aatgacggta cctacagaag aagcaacg                 48


<210> 33
<211> 37
<212> DNA

<213> Artificial Sequence

<220>
<223> Synthetic primer

<400> 33
agttcactgg acggtaactg acgctgaagt gcgaaag                                37

<210> 34
<211> 42
<212> DNA
<213> Artificial Sequence

<220>
<223> Synthetic primer

<400> 34
tagttggtga ggtaatggcc caccaagact atgatgttta gc                          42